# EUROPEAN PATENT SPECIFICATION

(11) **EP 3 725 374 B1**
(45) Date of publication and mention of the grant of the patent: **19.06.2024**
(21) Application number: 20176944.5
(22) Date of filing: 06.02.2018
(51) Int. Cl.: A61Q 17/04, A61K 8/44, A61K 8/67, A61K 31/197, A61P 17/10, A61P 17/06, A61Q 19/00, A61K 31/593, A61Q 19/08

(54) **SUNSCREEN COMPOSITION**
SONNENSCHUTZZUSAMMENSETZUNG
COMPOSITION D'ÉCRAN SOLAIRE

(30) Priority: 06.02.2017 NO 20170179
(43) Date of publication of application: 21.10.2020
(62) Divisional of application: 18704212.2
(73) Proprietor: 3Skin AS, 0487 Oslo (NO)
(72) Inventor: SOLER, Ana, 0487 Oslo (NO); WARLOE, Trond, 0487 Oslo (NO); PENG, Qian, 0487 Oslo (NO)
(74) Representative: Dehns

(56) References cited:
- EP-A2- 2 269 645
- WO-A1-84/02845
- WO-A2-2006/039281
- WO-A2-2013/092936
- JP-A- 2004 175 731
- US-A- 4 610 978
- US-A- 5 520 905
- US-A1- 2008 312 181
- US-A1- 2010 298 758
- US-A1- 2011 212 146
- US-A1- 2016 339 222
- DATABASE GNPD [Online] MINTEL; January 2016 (2016-01), "Suncoat Anti-Aging Sunscreen Cream SPF 60", XP002780623, Database accession no. 3630979
- DATABASE GNPD [Online] MINTEL; July 2016 (2016-07), "Instant Dry Mist Sunblock SPF 50", XP002780624, Database accession no. 4118341

## Description

### Background for the invention:

Skin forms the largest organ of the human body. Skin consists of 3 layers, namely the epidermis, dermis and subcutis. The epidermis is the uppermost, epithelial layer of the skin. It acts as a physical barrier, preventing loss of water from the body, and preventing entry of substances and organisms into the body.

The epidermis consists of stratified squamous epithelium, i.e. it consists of layers of flattened cells. Skin, hair and nails are keratinised, meaning they have a dead, hardened hydrophobic surface made of the protein keratin. The epidermis comprises three main types of cell, namely keratinocytes (skin cells), melanocytes (pigment-producing cells) and Langerhans cells (immune cells). The Merkel cell is a fourth, less prevalent, epidermal cell.

Keratinocytes mature and differentiate with accumulation of keratin as they move outwards. They form four or five distinct strata, which are (i) the Stratum corneum (horny layer) with dead, dried-out hard cells, (ii) the Stratum granulosum (granular layer) with cells containing basophilic granules and outwardly separated from stratum corneum by the thin stratum lucidum, (iii) the Stratum spinulosum (spinous, spiny or prickle cell layer) in which the cells become increasingly flattened as they move upward and (iv) the Stratum basale (basal layer) with columnar (tall) regenerative cells.

The basement membrane is a specialised structure that lies between the epidermis and dermis.

The dermis is the fibrous connective tissue or supportive layer of the skin. The major fibres are collagen fibres and elastin which are interwoven. The subcutis is the fat layer immediately below the dermis and epidermis. The subcutis mainly consists of fat cells (adipocytes), nerves and blood vessels. New epithelial skin cells are created in the skin's lower layer, the stratum granulosum. Over time, cells migrate to the surface of the skin and become more acidic. During their 30 day journey, they die and become saturated with keratin. Keratin and associated lipids are important because they protect the skin from outside elements.

Many factors may contribute to the deterioration in the cosmetic appearance of skin including disease, injury, environmental factors, age, hormone levels, medication, externally applied or ingested materials, genetic conditions or a combination of these and other factors. Age related deterioration in the cosmetic appearance of skin is a universal factor. This deterioration can be seen in irregularities or abnormalities in the skin, which may appear as, e.g. wrinkles and fine lines, thinning of the skin, dry skin, altered pigmentation, such as skin discoloration or hyperpigmentation, and/or loss of firmness and elasticity, i.e. increased laxity (sagging).

Photoageing, also known as dermatoheliosis, is a term used for the characteristic changes induced by chronic UVA and UVB exposure.

Many cosmetic compositions are marketed as capable of reducing the visible signs of aging. The effectiveness of anti-aging cosmetics depends in part on the active ingredient or ingredients contained therein. Commonly used ingredients in anti-aging cosmetics include antioxidants, exfoliants, moisturizing agents, proteins and peptides, enzymes and cofactors.

There remains, however, a continuing desire amongst consumers and cosmetic manufacturers to further improve skin care products, increasing and improving the benefits that can be delivered through application of a composition.

### Discussion of prior art

Prior art that provides background information and/or explains some of the putative mechanisms by which the compositions of the present compositions may exert their effects is discussed below. It must be appreciated that this prior art was either identified by the inventors with an inventive mind, or identified in a search carried out with knowledge of the invention. This discussion should therefore not be taken as an acknowledgement that any or all of these matters form part of the prior art base or were common general knowledge in the field relevant to the present disclosure as it existed before the priority date of each claim of this application. Moreover, the prior art was analysed by the inventors with an inventive mind, so this discussion does not reflect how an unimaginative person may analyse the prior art.

Photodynamic therapy (PDT) for cancer patients has developed into an important new clinical treatment modality in the past 25 years. PDT involves administration of a tumour-localizing photosensitizer or photosensitizer prodrug and the subsequent activation of the photosensitizer by light.

Typically, 5-aminolevulinic acid (ALA) is administered as a prodrug, which is metabolized to promote the accumulation of fluorescent protoporphyrin IX (PpIX). PpIX is an efficient photosensitizer.

PDT may be used to treat skin cancers, in which case the administration is typically topical. Commonly, a cream containing ALA is applied to the relevant skin region. This cream layer may be covered for an occlusion period, and the skin region is subsequently exposed to light, causing singlet oxygen-induced photodamage to the region. The amount of ALA in the cream is usually 5-20 wt%. For example, the known Levulan^{™} Kerastick^{™} (by DUSA Pharmaceuticals, Inc., 25 Upton Drive, Wilmington, MA 01887) contains about 20 wt% ALA. The known Ameluz^{™} gel (by Biofrontera of Hemmelrather Weg 201, D-51377 Leverkusen, Germany) contains about 10 wt% ALA.

The light source used in most cases is a laser, typically with a wavelength of approximately 630 nm.

PDT is a medical treatment which can have unpleasant side-effects, which may, e.g., involve redness and/or a tingling or burning sensation. Typically, for about 2 days after the ALA has been applied, the patient should take care to not expose treated areas to light. For example, the patient information provided in relation to Levulan^{™} Kerastick^{™} warns that patients should avoid exposure of the photosensitive treatment sites to sunlight or bright indoor light for at least 40 hours after application of Levulan^{™} Kerastick^{™}. It also states that exposure may result in a stinging and/or burning sensation and may cause erythema or oedema of the lesions. It warns that sunscreens will not protect against photosensitivity reactions caused by visible light.

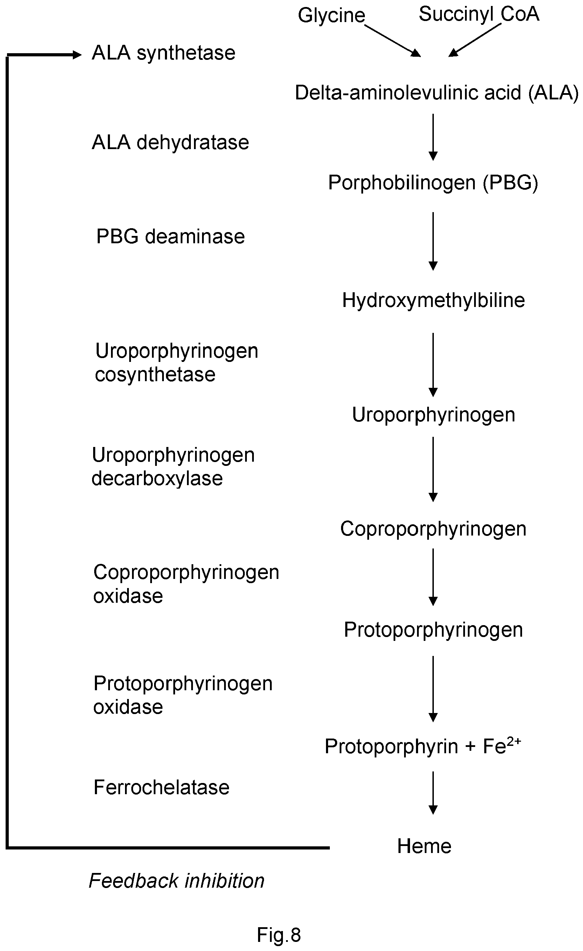

Thus, photodynamic therapy (PDT) is based on a light-activated photosensitiser including porphyrins, which is induced by 5-aminolevulinic acid (ALA). As illustrated in Figure 8, in the first step of the heme biosynthetic pathway, ALA is formed from glycine and succinyl CoA. Via a number of steps, ALA is ultimately converted into protoporphyrin IX. The last step of the heme biosynthesis pathway is the incorporation of iron into protoporphyrin IX (PpIX, a potent photosensitiser) and takes place in the mitochondria under the action of the rate-limiting enzyme, ferrochelatase. By adding exogenous ALA, the balance of the pathway may be disturbed and so the naturally occurring several porphyrins (PpIX in particular) may accumulate because of the limited capacity and/or low activity of ferrochelatase.

Porphobilinogen deaminase is another enzyme of the heme synthesis pathway. Its activity is higher in proliferative cells; while that of ferrochelatase is lower, so that porphyrins accumulate with a high degree of selectivity in these cells (Peng Q. et al., Cancer, 1997; 79:2282-308. Peng Q. et al., Photochem Photobiol, 1997; 65:235-51). This established modality is conventionally used for treating certain skin diseases and also significantly photodamaged skin conditions. Without wishing to be bound by theory, PDT is believed to act inter alia via cytotoxic Reactive Oxygen Species (ROS).

A PDT effect is dependent upon the PDT dose, which in turn is determined by the amounts of a photosensitizer and light dose. Generally, a high PDT dose generates a high ROS level to eliminate diseased cells/tissues; whereas a low PDT dose appears to produces a low ROS level which, according to one in vitro study, appears to stimulate cell proliferation (Blázquez-Castro A, et al., Photochem Photobiol Sci. 2014; 13:1234-40.

A study by Pan Q, et al., Investigative Ophthalmology & Visual Science. 2011; 52:1723-34 suggests that suggest that H2O2 at low levels promotes cell adhesion, migration, and wound healing in cornea cells or tissue. Day RM., et al., Dose Response. 2006; 3:425-42 also considered the effect of exogenous H2O2. Peplow PV, et al., Photomedicine and Laser Surgery. 2012; 30:118-48 reported that PDT improved healing outcomes in several animal wound models, but mainly had an inhibitory effect on cells *in vitro.*

Conventional sunscreen salves or compositions are based on the UV-blocking effect of diverse compounds ("Photoprotective efficacy and photostability of fifteen sunscreen products having the same label SPF subjected to natural sunlight." Hojerová J. et al., Int. J. Pharm. 2011), and such compounds are consequently combined with carriers to create sunscreen compositions to be applied onto the skin as a protective layer to avoid sunburn injuries and other damaging effects to the skin's cells from the sun's UV-radiation inter alia on the condition of the cells of the skin. If subjected to prolonged UV radiation skin cells may however transform into diseased states such as actinic keratoses, dysplasia, non-melanoma skin cancers and melanoma cancer.

US patent no. 5.520.905 proposed a cosmetic or dermatological preparation comprising 5-aminolevulinic acid (ALA) as an active ingredient to be used inter alia as a sunscreen composition. The only composition tested was a simple acrylate gel comprising 5% ALA.

Other prior art pertaining the addition of ALA and PpIX and Vit. D that is also mainly concerned with the photodynamic therapeutic action of such combinations is listed below.

Maytin, Edward V.; Anand, Sanjay; Atanaskova, Natasha; Wilson, Clara; Dept. of Dermatology, Lerner Res. Inst., Cleveland Clinic, Cleveland, OH, 44915, USA; "Vitamin D as a potential enhancer of aminolevulinate-based photodynamic therapy for nonmelanoma skin cancer"; Proceedings of SPIE (2010), 7551 (Optical Methods for Tumor Treatment and Detection: Mechanisms and Techniques in Photodynamic Therapy XIX.

Chen, Xiaofeng; Wang, Chunlei; Teng, Lei, Liu, Yaohua; Chen, Xin; Yang, Guang; Wang, Ligang; Liu, Huailei; Liu, Ziyi; Zhang, Dongzhi; Zhang, Yongxiang; Guan, Hongpeng; Li, Xianfeng; Fu, Changyu; Zhao, Boxian; Yin, Fei; Zhao, Shinguang; Dept. of Neurosurgery, The Fist Affiliated Hospital of Harbin Medical University, Harbin, Peop. Republ. Of China: "Calcitriol enhances 5-aminolvulinic acid-induced fluorescence and the effect of photodynamic therapy in human glioma"; Acta Oncologica (2014), 53(3), 405-413.

Anand, Sanjay; Hasan, Tayyaba; Edward V, Department of Biological Engineering (Lerner Research Institute) Cleveland Clinic, Cleveland, OH, USA; American Association for Cancer Research, "Mechanism of Differentiation - Enhanced Photodynamic Therapy for Cancer: Upregulation of Coproporphyrinogen Oxidase by C/EBP Transcription Factors", Molecular Cancer Therapeutics (2013), 12(8), 1638-1650.

Vallinayagam, Ramakrshnan; Weber, Joanne; Neier, Reinhard; Institut de Chimie, Universite de Neuchatal, CH-2009, Switzerland; American Chemical Society; "Novel bioconjugates of aminolevulinic acid with vitamins. Organic Letters (2008), 10(20), 4453-4455.

The enhancement of PpIX-production in skin cells by the simultaneous introduction of vitamin D and ALA to epidermis cells is known (Nobuyuki et al: "Vitamin D Enhances ALA-Induced Protoporphyrin IX Production and Photodynamic Cell Death in 3-D Organotypic Cultures of Keratinocytes", J. of Invest. Dermatology (2007) 127, 925-934). However, this effect was suggested in phototoxic cell killing in Vitamin D-enhanced photodynamic therapy and not as a way to create a sun blocking transdermal composition, and could be interpreted as pointing away from including ALA and Vit. D3 in combination to the skin in a composition to be added to the skin and subsequently subjected to radiation.

### Disclosure of the invention

The present invention provides compositions for use in treating or preventing skin damage from ultraviolet radiation, and kits, as defined in the appended claims.

In one aspect the present invention provides a composition for use in treating or preventing skin damage from ultraviolet radiation, said composition comprising
(a) 5-aminolevulinic acid (5-ALA) or an acid addition salt thereof;
(b) vitamin D3 (Vit. D3); and
(c) a UVA and/or UVB protective agent,
wherein said 5-ALA or acid addition salt thereof is present in an amount of 0.1 to 2.0 wt% of the composition, and wherein said vitamin D3 is present in an amount of 0.0001 to 0.005 wt% of the composition.

The compositions for use provided herein are active against skin damage from ultraviolet radiation (UV-radiation) and drying out, ageing, scar tissue formation and blemishes; while simultaneously blocking harmful UV (ultraviolet) radiation from sunrays and supplying Vit. D3 to the skin.

The term "derivative of ALA" is disclosed herein to mean a porphyrin precursor. A porphyrin precursor is any compound that when added exogenously to a subject leads to the accumulation of porphyrins, preferably PpIX. The porphyrin precursor preferably has structural and functional similarly to ALA. It may preferably be selected from an ALA salt, an ALA ester, or an ALA ester salt. The ester may be any dermatologically and/or pharmaceutically acceptable ester, e.g. the methyl ester or hexyl ester. Suitable ALA esters and salts are well known in the art, e.g. from WO2002010120, WO199101727, WO199628412 and WO 2005092838. The salt may be any dermatologically and/or pharmaceutically acceptable salt, e.g. the hydrochloride salt, sulfonic acid salt or sulfonic acid derivative salt, preferably the hydrochloride salt. Thus, it is preferably selected from ALA-HCl, ALA methyl ester HCl and ALA hexyl ester HCl.

The term "ALA" is used herein to refer to 5-aminolevulinic acid, unless otherwise stated it is also used as a convenient shorthand for a 5-aminolevulinic acid salt The ALA will typically be used in the salt form, so it should be understood that the salt, particularly the hydrochloride salt of ALA, is particularly preferred. Where it is explicitly desired to refer to 5-aminolevulinic acid, without encompassing a salt thereof, the term "free acid form of ALA" or "free ALA" is used.

The ALA is present in an amount of 0.1 to 2.0 weight percent (wt%) of the composition, or 0.1 to 1.0 wt%.

Thus, ALA may be present in an amount of at least 0.1 wt%, at least 0.11 wt%, at least 0.12 wt%, at least 0.13 wt%, at least 0.14 wt%, at least 0.15 wt%, at least 0.16 wt%, at least 0.17 wt%, at least 0.18 wt%, at least 0.19 wt%, at least 0.2 wt%, at least 0.25 wt%, at least 0.3 wt%, at least 0.4 wt%, at least 0.5 wt%, at least 0.6 wt%, at least 0.7 wt%, at least 0.8 wt%, at least 0.9 wt% or at least 1.0 wt%, and/or no more than 2.0 wt%, or no more than 1.5 wt%.

Alternatively viewed, it may be present in an amount of 2.0 wt%, about 1.5 wt%, about 1.0 wt%, about 0.9 wt%, about 0.8 wt%, about 0.7 wt%, about 0.6 wt%, about 0.5 wt%, about 0.4 wt%, about 0.3 wt%, about 0.2 wt%, or about 0.1 wt%.

By a "low dose" is meant no more than 2 wt%, no more than 1 wt%, or no more than 0.5 wt%. For example, it may be present in an amount of 0.1 to 1.0 wt%, 0.1-0.5, 0.1-0.4, 0.1-0.3, or 0.1-0.2 wt%.

Without wishing to be bound by theory, the inventors believe that a low dose of ALA may, when the skin is exposed to sunlight, stimulate one or more types of skin cells to proliferate/regenerate, which can result in a cosmetic improvement.

By a "medium dose" is meant herein no more than 2.0 wt%, and preferably more than 0.1 wt%, more than 0.2 wt%, more than 0.3 wt%, more than 0.4 wt%, more than 0.5 wt%, more than 0.6 wt%, more than 0.7 wt%, more than 0.8 wt%, more than 0.9 wt%, more than 1.0 wt%, more than 1.2 wt%, more than 1.5 wt%, more than 1.8 wt%, or more than 1.9 wt%. For example, it may be present in an amount of 1 to 2 wt%.

Without wishing to be bound by theory, the inventors believe that a medium dose of ALA may, when the skin is exposed to sunlight, inhibit, damage and/or kill one or more types of skin cells that are abnormally proliferative and/or inflamed.

The above passages refer to the amount of the ALA in the composition. It must be appreciated that if the ALA is present as a salt, which will preferably be the case, the amount of free ALA present in the composition will be less. For example, 100 mg of aminolevulinic acid hydrochloride is equivalent to 78 mg of free ALA. The wt% indicated herein is preferably the wt% of the hydrochloride salt of ALA. If a different salt of ALA is used, then it should be present in an amount that provides a corresponding amount of free ALA.

As mentioned above, for PDT ALA is typically present in an amount of 5-20 wt%, so this range is, in the prior art, considered to be therapeutic. A composition comprising a smaller amount of a porphyrin, porphyrin analogue, and/or precursor of a porphyrin or porphyrin analogue such as ALA may therefore be referred to as "sub-therapeutic". "Sub-therapeutic" is used herein to refer to an amount of no more than 2.0 wt%, preferably less than 1.5, or less than 1 wt%. Thus, the term "sub-therapeutic" is used herein to indicate that the amount is insufficient to be used effectively in conventional PDT. As will be apparent from the disclosure herein, the present inventors have determined that an amount of ALA of no more than 2 wt% can, however, be effective in the treatment of certain conditions such as actinic keratosis. Thus, it has surprisingly been determined that a "sub- therapeutic" amount of ALA can, in some embodiments, be therapeutically effective. It has also surprisingly been determined that a "sub-therapeutic" amount of ALA can, in some embodiments, be cosmetically effective, e.g. be effective in treating or preventing skin ageing.

Cholecalciferol is also known as Vitamin D3.

The vitamin D3 (Vit. D3) may be present in an amount of 0.0001 to 0.005% weight percent (wt%) of the composition. Preferably, the amount is at least 0.0002, 0.0003, 0.0004, 0.0005, 0.0006, 0.0007, 0.0008, 0.0009 or 0.001 and/or no more than 0.005, 0.004, 0.003 or 0.002 wt%. For example the Vit. D3 may be present in an amount of about 0.0001-0.0005 wt%, e.g. about 0.0001-0.0002 wt%.

The UVA and/or UVB protective agent may be any agent that provides protection for the skin against UVA and/or UVB when applied topically to the skin. Preferably, the composition may comprise 2 more such agents, e.g. a mixture of at least 3, 4, 5, or 6 of such agents, preferably comprising at least one UVA protective agent and at least one UVB protective agent.

The agent may be a broad-spectrum UV protective agent that is protective against both UVA and UVB. For example, it may be a broad-spectrum UV absorber with two absorption peaks, one at UVB (e.g. 303 nm), and one at UVA (e.g. 344 nm), such as Drometrizole trisiloxane (INCI), a lipophilic benzotriazole derivative.

The agent may, for example, be an agent that absorbs UVA and/or UVB. Alternatively and/or in addition, the agent may be an agent that reflects UVA and/or UVB. The UVA and/or UVB protective agent is preferably an organic agent, rather than an inorganic one. Preferably, the composition comprises little or no inorganic UVA and/or UVB protective agents.

An UVA and/or UVB protective agent may, e.g., be selected from a) p-aminobenzoic acids, their esters and derivatives (for example, 2ethylhexyl p-dimethylaminobenzoate); b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin; c) benzophenones (for example oxybenzone or dioxybenzone); d) dibenzoylmethanes, such as 4- (tert-butyl)-4'-methoxydibenzoylmethane; e) 2-phenylbenzimidazole-5 sulfonic acid and its salts; f) alkyl-ss, ss-diphenylacrylates, for example alkyl α-cyano-ss, ss-diphenylacrylates such as octocrylene; g) triazines, such as 2,4,6-trianilino-(p-carbo-2-ethyl-hexyl-1-oxi)-1,3,5 triazine; h) camphor derivatives, such as methylbenzylidene camphor; i) Salicylates, such as ethylhexyl salicylate, homosalate, or octyl salicylate; j) mixtures thereof. Other preferred UVA and/or UVB protective agents include those selected from the group consisting of Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15, Drometrizole Trisiloxane, Octyl Triazone, Bemotrizinol, Ecamsule and mixtures thereof. For example, a combination of Butyl Methoxydibenzoylmethane (Avobenzone), Octocrylene, Drometrizole Trisiloxane, Octyl Triazone, Bemotrizinol, and/or Ecamsule is preferred.

The UVA and/or UVB protective agent, or combinations thereof, may be present in an amount sufficient to make the composition effective as a sunscreen composition. The agent or combination of agents may be present in an amount from 0.1 to 50 wt% of the composition, e.g. at least 1, 2, 3, 4 or 5 wt% and preferably no more than 50, 40, 30, 25, 20, 18, 15, 12 or 10 wt%. For example, the composition may comprise about 1-10 different UVA and/or UVB protective agents, which may each independently be present in an amount of about 0.5 to 10 wt%.

For example, the composition may comprise Butyl Methoxydibenzoylmethane 5.0%w/w, Octocrylene 4.5%w/w, Drometrizole Trisiloxane 4.0%w/w, Octyl Triazone 2.5%w/w, Bemotrizinol 2.0%w/w, and/or Ecamsule 1.5%w/w.

Thus, the composition provided for use herein may be a sunscreen composition. It may also be a pharmaceutical composition.

The composition may optionally be formulated by combining (a) ALA or an acid addition salt thereof as defined herein; (b) the vit D3 as defined herein; and (c) a conventional sunscreen composition. The conventional sunscreen composition may be selected from any suitable sunscreen composition. Suitable examples include those mentioned elsewhere herein, La Roche-Posay Anthelios xl, Actinica Galderma 50+, and Cosmica 50.

By "sunscreen composition" is meant a composition comprising a sufficient amount of one or more UVA and/or UVB protective agents to protect human skin from the harmful effects of exposure to the sun, particularly UVA and/or UVB rays, when properly applied to said human skin. It is well known that the duration of protection varies depending *inter alia* on the type and concentration of UVA and/or UVB protective agents present in the sunscreen composition. Thus, the sunscreen composition may, e.g., have a sun protection factor (SPF) of at least 5, 10, 15, 20, 25, 30, 35, 40, 45, 50 or 100. Alternatively and/or in addition, it may have a UVA rating of at least one, 2, 3, 4 or 5 stars. An SPF of at least 15, 30 or 50 and a UVA rating of at least 4 or 5 is preferred.

As discussed below, the composition provided for use herein may be a transdermal composition and/or it may, e.g., be included in a transdermal carrier or carrier system.

With chronological age and/or exposure to adverse environmental factors, the visual appearance, physical properties, and physiological functions of skin change; thus, signs of skin ageing start to develop and progress. Skin can also have areas of dryness, scarring, and/or blemishes resulting from minor damage to the epidermis and/or dermis.

The invention provides the composition for use in treating and/or preventing skin damage from ultraviolet radiation and one or more of the signs of skin ageing, dryness, scarring, and/or blemishes. These may be referred to as cosmetic conditions affecting the cosmetic appearance of skin.

By "treatment and/or prevention of skin aging" is meant the treatment and/or prevention of one or more signs of skin ageing..

Thus, one or more signs of skin ageing (which may optionally be sun-induced skin ageing) may be reduced or eliminated, and/or the development or progression of one or more signs of skin ageing may be reduced or eliminated.

The signs of skin ageing may be selected from one or more of fine lines, wrinkles, reduced skin elasticity, thinning of the skin (i.e. reduction in skin density), reduction of skin firmness, reduction of colour evenness (tone), increase in surface texture coarseness, and/or increase in mottled pigmentation.

By "treatment and/or prevention of skin dryness" is meant that one or more signs of skin dryness may be reduced or eliminated, and/or the development or progression of one or more signs of skin dryness may be reduced or eliminated. The signs of skin dryness may be selected from a feeling of skin tightness, skin flaking, skin scaling, skin cracks, and/or skin redness. Preferably, skin dryness is reduced or eliminated, i.e. the moisture content of the skin is increased or restored to a normal level.

By "treatment and/or prevention of scarring and/or blemishes of skin" is meant that one or more signs of skin scarring and/or blemishes may be reduced or eliminated, and/or the development or progression of one or more signs of scarring and/or blemishes may be reduced or eliminated.

The signs of skin scarring and/or blemishes may be selected from keloid scars or hypertrophic scars, each typically characterised by raised tissue; contracture scar typically characterised by tight skin; pitted scars; and/or grooved scars.

The change may be at the level of the dermis, the epidermis, or both.

Preferably, the composition for use has the purpose and/or effect of increasing the elasticity of the skin and/or increasing the density of the skin, most preferably both. The increase may be at the level of the dermis, the epidermis, or both.

The present invention also encompasses a kit comprising:
(a) 5-aminolevulinic acid (5-ALA) or an acid addition salt thereof;
(b) vitamin D3; and
(c) a UVA and/or UVB protective agent,
wherein said kit comprises a container comprising a composition comprising said 5-ALA or acid addition salt thereof in an amount of 0.1 to 2.0 wt% of the composition, and a separate container comprising a composition comprising said vitamin D3 present in an amount of 0.0001 to 0.005 wt% of the composition.

The kit may include a container with a solution of the ALA or an acid addition salt thereof as defined herein included in a carrier or carrier system, said kit also including a separate container with the Vit. D3 as defined herein included in a comparable or the same carrier or carrier system, wherein administration of an amount of the composition from each container could be performed for obtaining the effects disclosed supra. In such an embodiment the kit should preferably contain application and use instructions in a mechanical (leaflet) or electronic form.

Thus, also provided is a kit comprising or consisting of (i) ALA or an acid addition salt thereof as herein defined; (ii) vitamin D3 as defined herein; and (iii) a UVA and/or UVB protective agent as defined herein; and optionally instructions on how to use the kit.

By "pharmaceutically acceptable" or "dermatologically acceptable" is meant that the compound, salt or other ingredient must be suitable for cosmetic and/or pharmaceutical applications and compositions. The ingredient also must be compatible with other ingredients in the composition as well as physiologically acceptable to the recipient.

As referred to herein, "cosmetic" is intended to refer to a treatment which does not cure, treat or prevent a medical disease or disorder, but instead serves as a skincare product to modify or improve the appearance of the skin, e.g. the colour, texture, elasticity, density or moisture content of the skin.

The term "transdermal" is sometimes used in the art to refer to compositions that penetrate the skin such that they reach the systemic circulation. However, in the context of the present invention the term "transdermal" is used to mean that the composition penetrates the skin, without implying or necessitating the reaching of the systemic circulation. By "transdermal" is meant herein that the composition penetrates and can exert its effects within at least the epidermis, but preferably also the dermis. Thus, a local epidermal and/or dermal effect can be achieved. Preferably, the transdermal composition can penetrate the basement membrane. Penetration into and/or beyond the hypodermis is not required.

The "subject" to which the compositions may be applied or administered may be any subject having an epidermis, particularly a mammal. The mammal may, e.g., be selected from a primate, domestic animal, livestock, and/or laboratory animal, particularly humans, mice, rats, rabbits, guinea pigs, cats, dogs, monkeys, pigs, cows, goats, sheep and/horses. Preferably, the subject is a human.

Optionally, the subject does not have skin cancer. Optionally, the subject does not have cancer (of any type). Optionally, the subject does not suffer from Porphyria. In embodiments where the composition is used to treat and/or prevent the signs of skin ageing, the subject optionally does not suffer from actinic keratosis and/or any of the dysplastic conditions mentioned elsewhere herein.

The composition is for use in treating and/or preventing skin damage from UV radiation, and the subject may be a subject who is suspected of having such a skin condition, a subject who is suspected of being at risk of developing such a skin condition, a subject who has been diagnosed with such a skin condition, a subject who has been diagnosed as being at risk of developing such a skin condition, a subject who suffers from such a skin condition, or a subject who has previously suffered from such a skin condition.

The subject may have skin that is aged, blemished and/or scarred skin. "Aged skin" refers to skin that displays one or more signs or symptoms of ageing, i.e. the appearance of wrinkles, fine lines, hyperpigmentation, laxity (sagging), dry skin, scaling or transepidermal water loss (TEWL). In particular, "aged skin" is determined relative to normal optimum skin, i.e. healthy, hydrated, normally pigmented and non-aged skin. In this respect, aged skin need not be related to the age of the subject and may be aged prematurely, e.g. by chronic exposure to sunlight (photo-damage). Thus, the relative parameters for "normal optimum skin" may be determined as the average measurements of the above signs of ageing from a number of subjects of the same or similar age to the subject in question, e.g. subjects that have not received chronic exposure to sunlight. Alternatively, the relative parameters for "normal optimum skin" may be taken as the measurements from subjects that are younger than the subject in question. In other words, the compositions may be for use in treating or preventing skin damage from ultraviolet radiation and and restoring the youthful appearance of skin, relative to the skin of the subject at an earlier age.

The compositions may be for use in treating or preventing skin damage from ultraviolet radiation and reducing and/or eliminating the signs of ageing, dryness, scarring, and/or blemishes. By "reducing" is meant that there is a decrease in one or the relevant signs in the treated skin during/after the treatment compared to before treatment/an earlier stage of treatment, or compared to treatment with a control or no treatment. The decrease may be qualitative and/or quantitative.

As shown in the Examples, the inventors have shown that the compositions provided for use herein are effective in the treatments disclosed herein. The effectiveness of the compositions/treatments may be assessed by examination of the relevant skin area prior to, during and/or after treatment, and/or by comparison to a control.

Thus, the skin during or after the treatment may be compared to (i) skin before the treatment; (ii) skin at an earlier stage of the treatment; (iii) an untreated control area of skin; and/or (iv) an area of skin treated with a control. The comparison may be to skin of the same subject or of a different subject. By "skin at an earlier stage of treatment" is meant an earlier time point. For example, skin after 3 weeks of treatment may be compared to skin after 1 week of treatment, in which scenario 3 weeks is a later time point and 1 weeks is an earlier time point.

For example, an area of skin treated with a composition provided for use herein may be compared to an area of skin of treated with a control composition. The skin treated with a control composition may be skin of a different subject, but is preferably skin of the same subject. Suitable control compositions include any conventional sunscreen compositions that do not comprise ALA and vitamin D3 in the amounts indicated herein.

The assessment may, e.g. be, or include, a visual assessment of the subject and/or of a suitable photograph of the subject. The assessment may, e.g. be carried out by a medical expert, such as a dermatologist. The assessment may be, or include, a self-assessment, e.g. the subject may report on the feeling and/or appearance of his/her skin, e.g. by answering a questionnaire and/or keeping a regular record.

Ultrasound may be employed to measure the thickness and/or elasticity of the skin, particularly in the bilateral temple areas. A suitable ultrasound device is one by Derma Lab, Skin Lab Combo, Cortex Technology, 9560 Hadsund, Denmark.

A photodynamic effect may be assessed using spectrophotometry, e.g. as discussed in relation to the Examples and Figures. However, it is believed that spectrophotometry may primarily show high fluorescence where a photodynamic anti-proliferative or cell-killing effect is taking place, so spectrophotometry may not be suitable for assessing a photodynamic effect in a cosmetic treatment scenario.

Clinical Grading of AK may be carried out according to the Olsen classification 1991.

"Administration" of the composition provided for use herein will typically be topical application of the application to the skin of the subject. The application of the composition to the skin of a subject may be in accordance with standard sunscreen application protocols. Thus, the composition should be applied topically to the skin. It may preferably be applied as a thin layer, e.g. a thickness of 0.1-5 mm, preferably about 1-2mm. Preferably, it should be applied a uniform manner. It may be applied to the skin of the whole body, although any application on or near mucous membranes such as the eyes, nostrils, mouth, and genitals should be avoided. Alternatively, it may be applied to one or more selected skin areas. For example, it may preferably be applied to any skin areas that are exposed to light, preferably sunlight, and/or will be exposed to such light within the next 24, 12, 10, 5, 2, or 1 hours, or 50, 40, 30, 20, 10, 5, 4, 3, 2 or 1 minutes.

Exemplary skin areas to which the composition may be applied include one or more of the face, head (if not covered by hair), neck, shoulders, cleavage, hands, arms, feet, legs, and torso. The face, neck and/or hands are particularly preferred. The application may be to one or more areas of the skin that show signs of, or are at risk of developing, fine lines and/or wrinkles, reduced elasticity, thinning, dryness, discoloration, hyperpigmentation, scarring, blemish, and/or laxity. The composition may, e.g., be applied exclusively to such a skin area, or preferentially to such a skin area.

The application may be carried out at regular intervals or be determined by actual or potential exposure to sunlight. For example, it may be daily, twice daily or three times daily.

Conveniently, the application need not be carried out by a medical professional. It may be carried out by a lay person, e.g. by the subject.

Without wishing to be bound by theory, the inventors believe that the amount of ALA-induced porphyrins is dependent upon a proliferative rate of cells. Since the proliferation rate of dysplastic keratinocytes in the photodamaged skin conditions is high, topical application of ALA can induce the dysplastic keratinocytes to produce a high amount of ALA-induced porphyrins and subsequently a high photodynamic effect on eliminating the dysplastic keratinocytes. On the other hand, the proliferation rate of resting stem cells and normal/regressive keratinocytes is relatively low and these cells produce a low amount of ALA-induced porphyrins and thus a low photodynamic effect on stimulating cell proliferation to induce skin regeneration (rejuvenation).

The composition for use of the present invention comprises vitamin D3 as defined herein. Vitamin D3 (Vit D3) is a hormone essential for normal bone and cardiovascular health. Calcitriol (1,25 dihydroxyD3) is the active form of the hormone. There are reports that calcitriol is a potent inducer of protoporphyrin IX (PpIX) in skin keratinocytes grown in organotypic cultures; and that Vit D3 has the ability to enhance PpIX levels within skin tumours *in vivo.* (Maytin EV. et al., Proceedings of SPIE, 2010; 7551. Chen X. et al., Acta Oncologica, 2014; 53:405-13. Anand S. et al., Molecular Cancer Therapeutics, 2013; 12:1638-50). A sunscreen in general reduces harmful UV radiation from sunrays, but it also reduces natural vitamin D3 (Vit. D3) synthesis in the skin because Vit. D3 synthesis requires UV radiation. By external addition of Vit. D3 to the skin in the form of a topically applied composition, Vit. D3 is readily assimilated through the skin to stimulate the proliferation of resting stem cells and normal keratinocytes in the skin. Without wishing to be bound by theory, the present inventors postulated that Vit. D3-mediated proliferative stem cells and normal keratinocytes together with fibroblasts may selectively take up ALA to produce porphyrins to induce a low photodynamic effect.

As mentioned above, a subtherapeutic (low) dose of ALA (e.g. less than 2 wt%) producing a photodynamic effect together with Vit. D3 can surprisingly both induce cell proliferation in an at least additive or even synergistic manner.

Thus it is considered to be an advantage if Vit. D3 could be transmitted through the skin to alleviate this creation of potential Vit. D3 reduction and deficiency through the use of conventional sunscreen compositions. In this regard, without wishing to be bound by theory, the compositions provided for use herein are believed to provide vitamin D3 to the skin (epidermis and/or dermis).

It is also known that transdermal sunscreens with UVA/UVB filters and also therapeutic doses of ALA may destroy some sun-damaged skin cells (keratinocytes) when exposed to the sun-light that contains red and blue light (so-called day-light photodynamic treatment). Additionally, the natural production of Vit. D3 in the skin when subjected to sun-light, will be inhibited by the addition of UV-blocking substances through a transdermal sunscreen. Thus, it is considered to be an advantage if Vit. D3 could be transmitted through the skin to alleviate this creation of potential Vit. D3 reduction and deficiency through the use of conventional sunscreen compositions.
Although the sunscreen composition for use according to the present invention blocks UVA and UVB, it does not block the light activating porphyrins for the photodynamic effects.

Such a combination for use according to the present invention utilizes the different degrees of ALA-induced porphyrin synthesis in various skin conditions (proliferative dysplastic cells versus resting stem cells and normal keratinocytes) to simultaneously generate surprisingly additive/synergistic effects on (1) reducing photodamaged skin conditions (2) inducing skin rejuvenation in addition to (3) Vit. D3 (or its analogues/derivatives) supplement for sunscreen-induced Vit. D3 reduction/deficiency.

As mentioned supra the composition provided for use herein, may be contained in a transdermal carrier or carrier composition or carrier system. Such a carrier or carrier composition may comprise conventional carrier(s) of a lipophilic nature or of emulsions including carriers of a hydrophilic nature combined with lipophilic foundations in a micelle system. Examples of such systems include various formulations of anhydrous bioadhesive creams which can be, for example, prepared with methylvinylether-maleic anhydride and poly(ethyleneglycol) or glycerol. Such compositions can also be solidified by poly(acrylic acid) to form some sorts of gel or other convenient administration form (se supra) as desirable. The formulations can in particular embodiments also be created as nanoemulsions and nanoparticles including ALA dendrimers. In addition or alternatively, a physical technique may be used to disperse ALA powder in an emulsifying ointment. Such a physical technique may be sonication and/or vigorous stirring, optionally together with physiologically acceptable adjuvants and/or emulsifiers.

Another consideration to take into account is the possible and unpredictable effect(s) that the constituents/ingredients of a composition comprising two or more substances might have on each other. Such effects might arise either by the substances acting or reacting with or on each other or the other ingredients of the combination (e.g. excipients and/or carriers) effecting unexpected detrimental effects on the tissues that the substances are added to, although each of the substances in the combination separately do not provide any such effects. Such effects may also arise during long-term storage of combinations since the ingredients of such combination may be attacked and/or changed by external factors, e.g. through the action of UV-light, reactive oxygen species carried by the surrounding air, microorganisms breaking down or otherwise changing the reactive species of the combinations, etc.

The permeability of the skin is associated with its general water-impermeability. When forming transdermally acting compositions it is normally desired to form them on a lipid-based foundation. Into this consideration enters the desire or necessity to include hydrophilic compounds into a lipophilic foundation or vice versa. However, systems for overcoming this problem exist e.g. by forming emulsions (oil-in-water or water-in-oil), micelles (e.g. through ultrasound action on oil-in-water or water-in-oil compositions or by rapid and/or vigorous agitation of the relevant mixtures), by using carriers for the relevant compounds where such carriers have a better solubility in the relevant system or combinations of such methods. Such methods may also include the addition of surfactants or detergents to such systems, which again suffers from the limitations of intra- or inter-effects mentioned supra. It is of importance, though, that the inclusion of surfactants, detergents, carriers, etc. do not exert any or very little detrimental effects on the active substances (ALA, Vit. D3) of the composition (see supra).

Possible administration forms that the compositions for use according to the present may exist in are, as well as lotions, salves, creams, gels, ointments, sticks, sprays, films, aerosols, drops, foam and/or mousse topical administrations forms, solutions, emulsions, suspensions, dispersions e.g. non-ionic vesicle dispersions, milks, patches, and any other conventional cosmetic forms in the art.

Processes for making transdermal compositions such as salves, lotions, lubrication, tinctures, creams, lotions, oils, pomades, foams, mousses, etc. are known as standard methods.

Ointments, gels and creams may, for example, be formulated with an aqueous or oily base with the addition of suitable thickening and/or gelling agents. Lotions may be formulated with an aqueous or oily base and will, in general, also contain one or more emulsifying, dispersing, suspending, thickening or colouring agents. Drops and solutions may be formulated with an aqueous or non-aqueous base also comprising one or more dispersing, solubilising or suspending agents. Aerosol sprays are conveniently delivered from pressurised packs, with the use of a suitable propellant.

The composition provided for use herein may optionally comprise one or more skin conditioning agents, preservatives, skin penetration enhancers, thickeners, viscosity modifying agents, gelling agents, sequestering agents, pH adjusting agents, waxes, drying agents, anti-itch agents, anti-foaming agents, buffers, neutralizing agents, colouring agents, discolouring agents, emulsifying agents, emulsion stabilizers, odorants, antioxidants, diluents, carriers, and/or propellants.

Preferably, the composition for use does not contain an anaesthetic agents, such as an agent selected from lignocaine, mepivacaine, bupivacaine, etidocaine, prilocaine, tetracaine, procaine, cocaine, and/or chloroprocaine. More preferably, it does not contain any agent selected from this group, most preferably it contains no anaesthetic agents at all.

Since the active substances being present in the compositions for use according to the invention could be administered by non-skilled individuals, the amount of composition per administration should be observed or monitored. This could be done by including an information leaflet or administration instructions together with the container with the relevant composition stating the advisable amount and/or number of times an administration should be conducted. Thus, the composition may be included in a container, pack, or dispenser together with instructions for administration.
Other means for ensuring that the recommended and/or advisable amount of the composition for use according to the invention is administered, is to present the composition in a container with a metered dispenser, e.g. with a pump delivering a certain amount of composition per pumping action. This may be combined with information about the number of times the pumping action is to be conducted. Such information may also present alternatives to where on the body the relevant composition is to be applied.

A typical recommendation is to apply 2 milligrams of sunscreen for every square centimeter of skin (2 mg/cm²), so the metered dispenser may be configured to dispense an appropriate amount. For example, about 1-5 mg or ml of the composition should ideally be applied to the area of the face and neck, so the metered dispenser may be configured to dispense e.g. about 1, 2, 3, 4 or 5 mg or ml of the composition per action.

In one embodiment the present invention relates to a kit including separate containers of a composition of ALA in the concentration range as specified supra and a container including the Vit. D3 composition in the concentration range as specified supra, the kit also comprising a UVA and/or UVB protective agent. Each of the compositions may be held in a container supplied with a metered pump. The composition in each container may be present in the form of a cream. The dispenser of the container/bottle of Vit. D3 cream may be adjusted to deliver 250 µg of Vit. D3 with each pumping action, also called the amount needed of the substance, when topically applied, to substitute one hour of dermal production of Vit. D by sun exposed skin. That represents 0,025% in 1 ml (g). If it is presumed that an average adult (80 kg body weight) is using 10 ml (g) of a sunscreen composition for use according to the present invention daily, this quantity of Vit. D should be diluted up to 10 g and then the concentration should be 0,0025% (w/w). A total substitution should not be needed. On the other hand if the sunscreen is applied only in the face (1g) the amount of Vit. D within the 0,0025% cream represents only about 1/10 of the "daily need". It should be observed that an excess of Vit. D on the skin is not necessarily absorbed through the dermis, but may lead to irritation of the skin. In addition, 15 minutes of sun exposure may be enough to cover the daily need of Vit. D.

Normally skin areas subjected to continuous sunray exposure (e.g. the face) could be applied more sunscreen than skin areas covered by clothing. Also the geographical location of the user as well as the meteorological weather forecast could be taken into consideration when administering the composition. In one embodiment it is conceivable that it will be possible to combine the use of the composition with an electronic information application being provided together with the acquisition of the container with the composition for use according to the invention. Such an application could in one alternative provide information pertaining application of the composition to children and to adults as well as information about the recommended dosage/amount of the composition relating to the state of the skin onto which it is to be applied, e.g. fair skin as opposed to coloured skin, undamaged as opposed to damaged skin, etc. Such an electronic application could in one embodiment e.g. be linked to the local weather-forecast in the relevant geographical area.

Upon application of such a combination to the skin as a thin layer, perspiration from the body may be emulsified into the ointment to dissolve the ALA. One of the major purposes for using such formulations is to stabilize ALA for a period of up to 6 months. Consequently, such formulations will improve the storage stability and shelf life of the relevant products.

In the disclosure, when specific numbers are given pertaining concentrations, amounts, volumes, etc. such numbers should be interpreted within the rules for standard deviation or at least with a measurement error of ± 10% of the specified number. As an example if a weight is specified to be 1,00 g, the error would be 0,1 g. Also (unless otherwise stated) all of the percentage numbers relate to percentages by weight calculated on the final weight of the composition, i.e. the weight sum of the ingredients in the composition.

The effect of the compositions for use according to the invention upon the skin is illustrated through the results presented in the following figures, wherein:

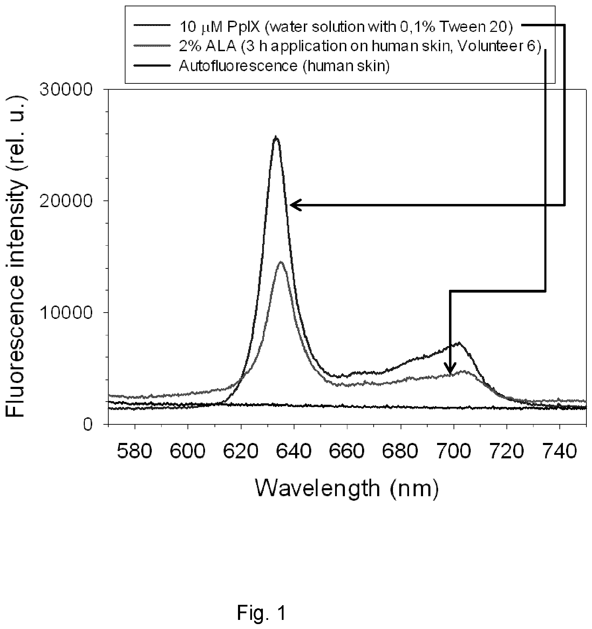

Figure 1 shows the wavelength peak in nm of the ingredients in a composition according to the present invention including 2% ALA (as the hydrochloride salt) (at 3-hours after application on human skin) or 10 µM) PpIX (corresponding to 0.56 mg% (w/v) PpIX) measured as the maximum relative fluorescence intensity versus a range of wavelengths spanning 580 - 740 nm. The measurements were conducted on separate compositions including: I) 10 µM PpIX (water solution with 0.1% (w/w) TWEEN 20); II) 2% (w/w) ALA (3-h application on human skin) and III) as a control autofluorescence of human skin without ALA application. Figure 1 shows a peak at a wavelength range of 620-650 nm, with a clear maximum peak at 635 nm, being the wavelength selected for conducting measurements in the subsequent tests;

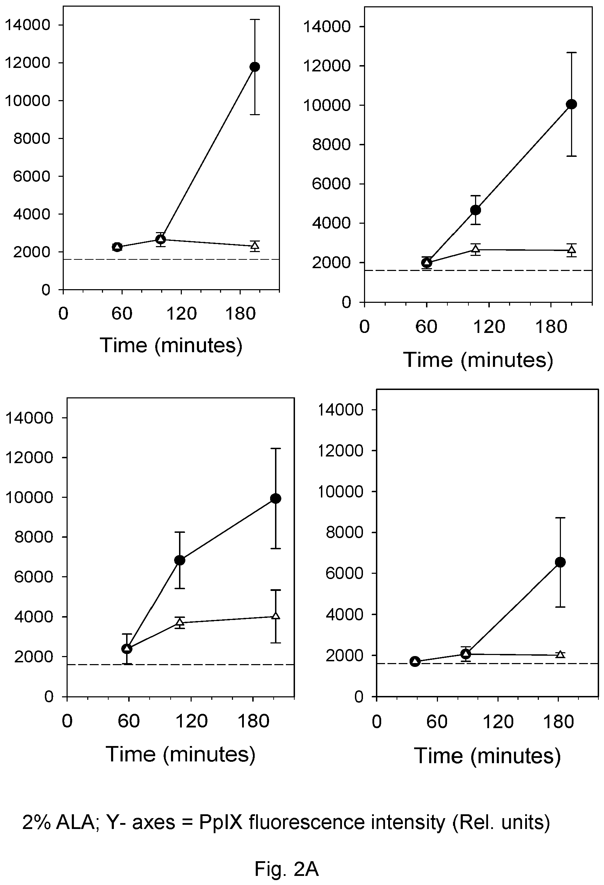

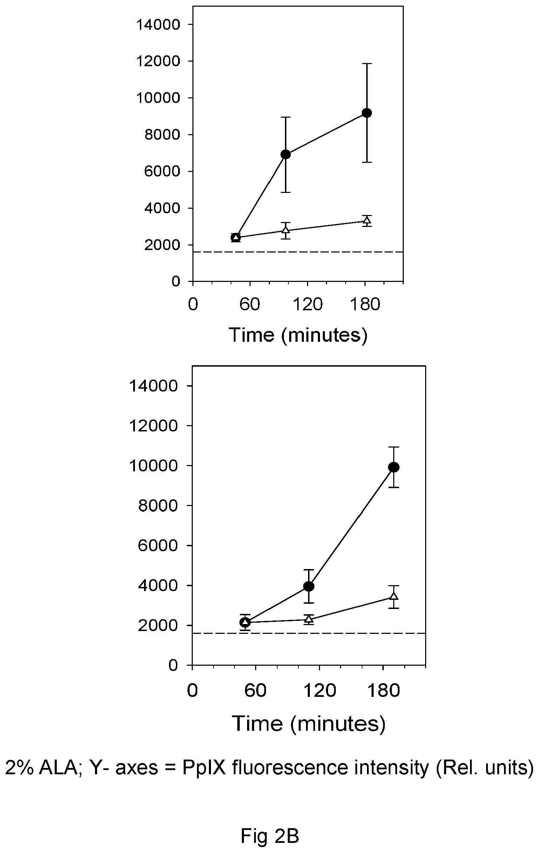

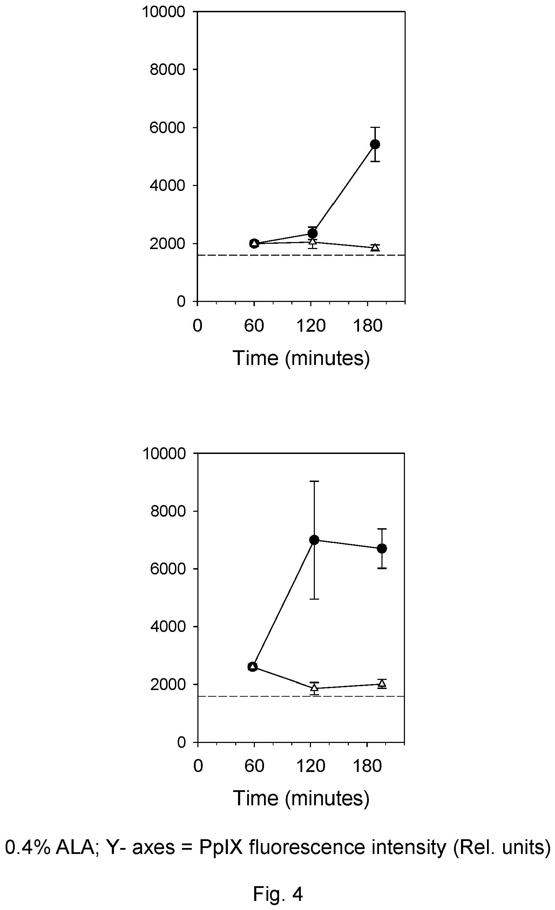

Figures 2 to 7 show PpIX fluorescence intensities as a function of time after topical facial application of ALA at various concentrations (as indicated) in 6 volunteers (Figs 2, 3, 7) and 2 volunteers (Figs. 4-6) of both sexes (male/female). The filled circles indicate the measurements at the photodamaged skin areas; while the unfilled cycles are normal skin areas; Thus,
Figure 2, i.e. Fig 2A and Fig.2B, shows PpIX fluorescence intensities as a function of time after topical facial application of 2% ALA in 6 different volunteers, where the filled circles indicate the measurements at the photodamaged skin areas; while the unfilled cycles are normal skin areas;
Figure 3, i.e. Fig 3A and Fig. 3B, shows PpIX fluorescence intensities as a function of time after topical facial application of 0.5% ALA in 6 different volunteers, where the filled circles indicate the measurements at the photodamaged skin areas; while the unfilled cycles are normal skin areas;
Figure 4 shows PpIX fluorescence intensities as a function of time after topical facial application of 0.4% ALA in 2 different volunteers, where the filled circles indicate the measurements at the photodamaged skin areas; while the unfilled cycles are normal skin areas;
Figure 5 shows PpIX fluorescence intensities as a function of time after topical facial application of 0.3% ALA in 2 different volunteers, where the filled circles indicate the measurements at the photodamaged skin areas; while the unfilled cycles are normal skin areas;
Figure 6 shows PpIX fluorescence intensities as a function of time after topical facial application of 0.2% ALA in 2 different volunteers, where the filled circles indicate the measurements at the photodamaged skin areas; while the unfilled cycles are normal skin areas;
Figure 7, i.e. Fig 7A and Fig. 7B, shows PpIX fluorescence intensities as a function of time after topical facial application of 0.12% ALA in 6 different volunteers, where the filled circles indicate the measurements at the photodamaged skin areas; while the unfilled cycles are normal skin areas; and
Fig. 8 shows information regarding the heme pathway.

### Examples:

In all of the Examples, ALA was used in the hydrochloride salt form (ALA-HCl) and the wt% indicated is the wt% of ALA-HCl, rather than the wt% of free ALA.

### Example 1.

The example relates to a sun-blocking composition according to the present invention comprising of ALA in a concentration of 0.12 % (w/w) (calculated on the basis of the total weight of the composition). The ALA together with Vit. D3 was included in a carrier composition of methylvinylether-maleic anhydride and poly(ethyleneglycol) included in a water solution with 0.1% (w/w) TWEEN 20. (La Roche-Posay Anthelios xl )

### Example 2.

This example concerns a composition like the composition of Example 1, but with an ALA concentration of 0.2 % (w/w).

### Example 3.

This example concerns a composition like the composition of Example 1, but with a ALA concentration of 0.3 % (w/w).

### Example 4.

This example concerns a composition like the composition of Example 1, but with a ALA concentration of 0.4 % (w/w).

### Example 5.

This example concerns a composition like the composition of Example 1, but with a ALA concentration of 0.5 % (w/w).

### Example 6.

This example concerns a composition like the composition of Example 1, but with a ALA concentration of 2.0 % (w/w).

In the compositions relating to Examples 1 - 6 the Vit. D3 was present at a fixed concentration of 250 µg/g. The active ingredients were included in a carrier composition (La Roche-Posay Anthelios xl ). Example 6 was conducted in the same manner as Example 1.

These studies relating to Examples 1 - 6 were aimed at finding out an optimal ALA concentration to generate sub-therapeutic PpIX-mediated photodynamic effects with no acute phototoxic reactions. The carrier creams containing ALA and Vit. D3 were topically applied to the human facial skin for 3 hours, during which the ALA-PpIX fluorescent signals were clearly detected by spectrophotometry as discussed in relation to Figure 1. The facial skin was then subjected to sun-exposure for a time of 120 minutes. The results in all 6 tested volunteers show a clear improvement of the skin conditions such as increased suppleness of the treated skin, reduced scar-tissue formation of the treated skin at the locations where the compositions had been applied, as assessed by medical experts visually at 1-, 4- and 8-week afterwards.

### Example 7.

This example relates to the results shown in Figure 2 concerning a test conducted with the La Roche - Posay Anthelios x1 conventional sunscreen composition including the ingredients 2% (w/w) ALA applied to the facial skin of 6 volunteers for 3 hours, during which time the ALA-PpIX fluorescent signals at the photodamaged skin areas of all 6 volunteers were clearly detected by spectrophotometry. Some ALA-PpIX fluorescent signals were also detected at the normal skin areas.

### Example 8

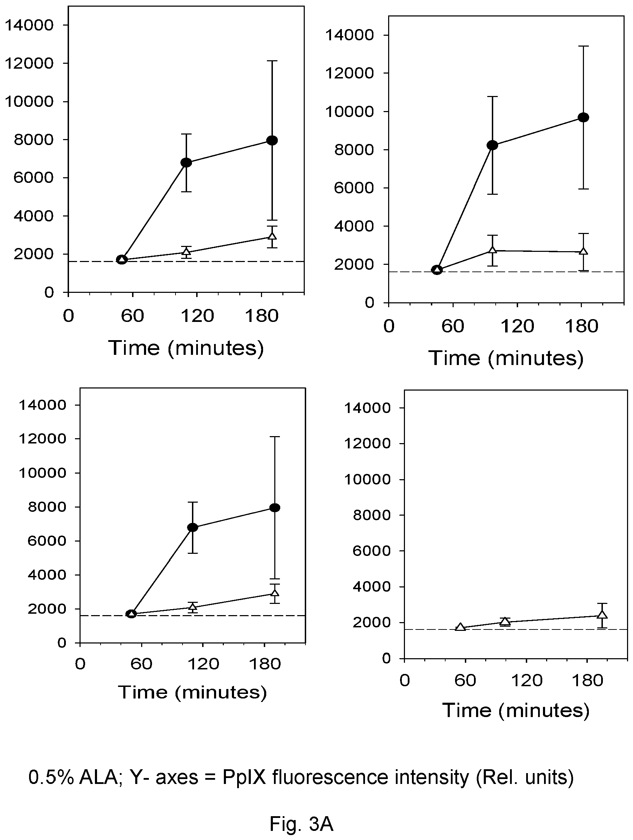

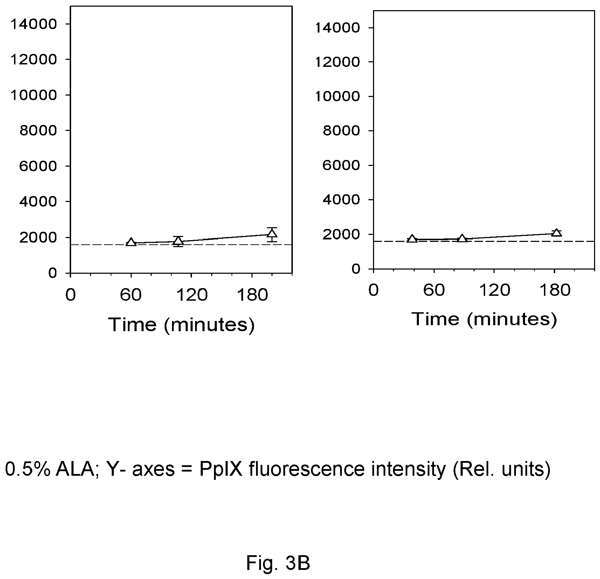

This example is relating to the results presented in Figure 3, and relates to a test conducted with the La Roche-Posay Anthelios xl sunscreen including the ingredient ALA/PpIX at 0.5% (w/w) applied to the facial skin of 6 volunteers for 3 hours, during which the ALA-PpIX fluorescent signals at the photodamaged skin areas of 3 volunteers were clearly detected by spectrophotometry. Some ALA-PpIX fluorescent signals were also detected at the normal skin areas.

### Example 9.

This example relating to the results shown in Figure 4, relates to a test conducted with the La Roche-Posay Anthelios xl sunscreen including the ingredient 0.4% (w/w) ALA/PpIX applied to the facial skin of 2 volunteers for 3 hours, during which the ALA-PpIX fluorescent signals at the photodamaged skin areas of the two volunteers were clearly detected by spectrophotometry. Some ALA-PpIX fluorescent signals were also detected at the normal skin areas.

### Example 10.

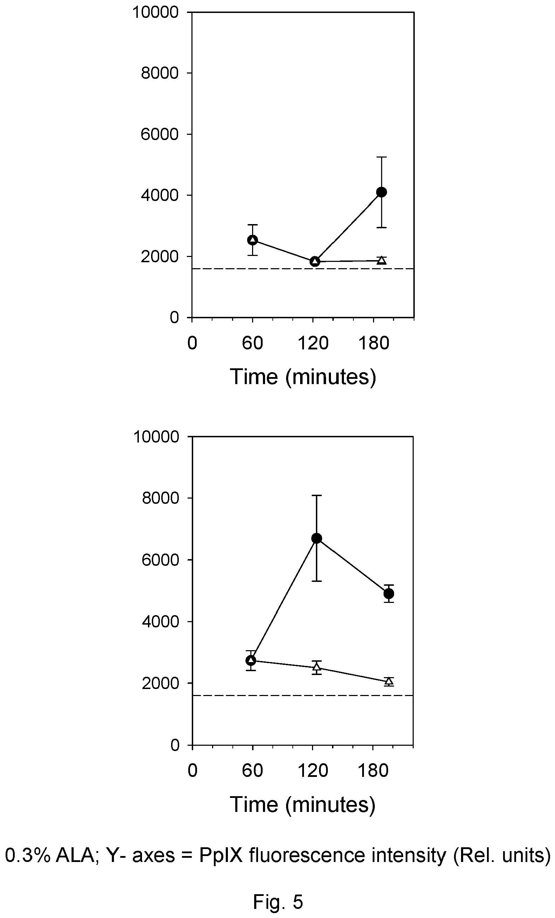

This example relates to the results shown in Figure 5 and relates to a test conducted with the La Roche-Posay Anthelios xl sunscreen including the ingredient 0.3% (w/w) ALA/PpIX applied to the facial skin of 2 volunteers for 3 hours, during which the ALA-PpIX fluorescent signals at the photodamaged skin areas of the two volunteers were clearly detected by spectrophotometry. Some ALA-PpIX fluorescent signals were also detected at the normal skin areas.

### Example 11.

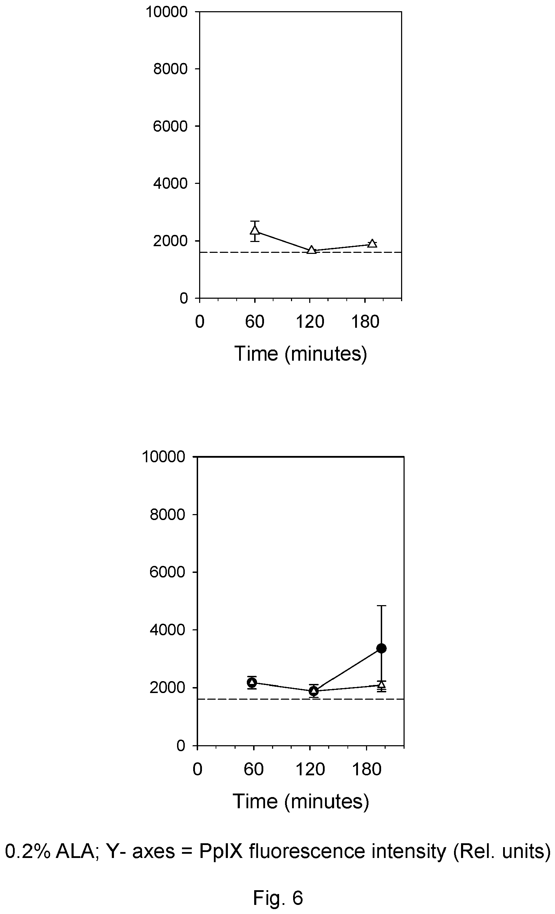

This example relates to the results shown in Figure 6 and relates to a test conducted with the La Roche-Posay Anthelios xl sunscreen including the ingredient ALA/PpIX 0.2% (w/w) applied to the facial skin of 2 volunteers for 3 hours, during which the ALA-PpIX fluorescent signals at the photodamaged skin areas of one of the two volunteers were clearly detected by spectrophotometry. Some ALA-PpIX fluorescent signals were also detected at the normal skin areas.

### Example 12.

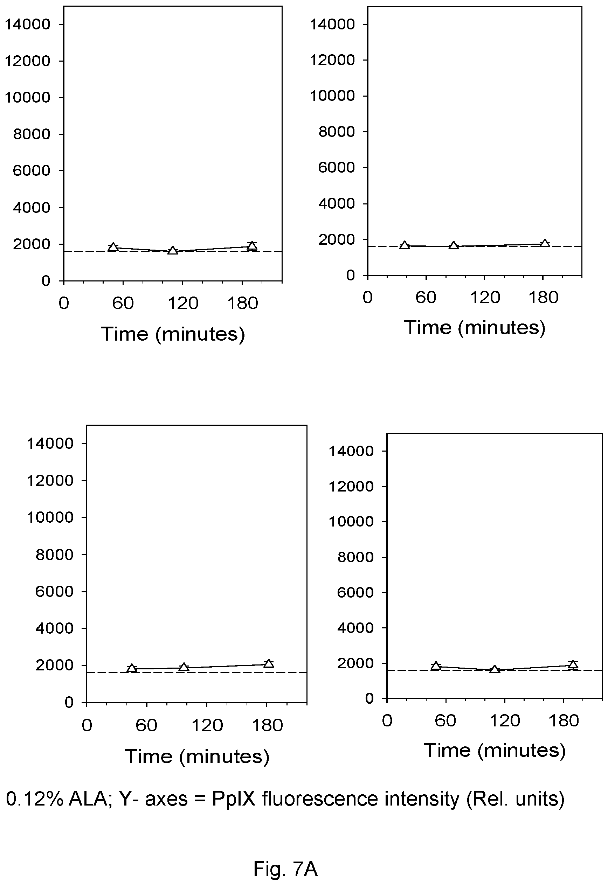

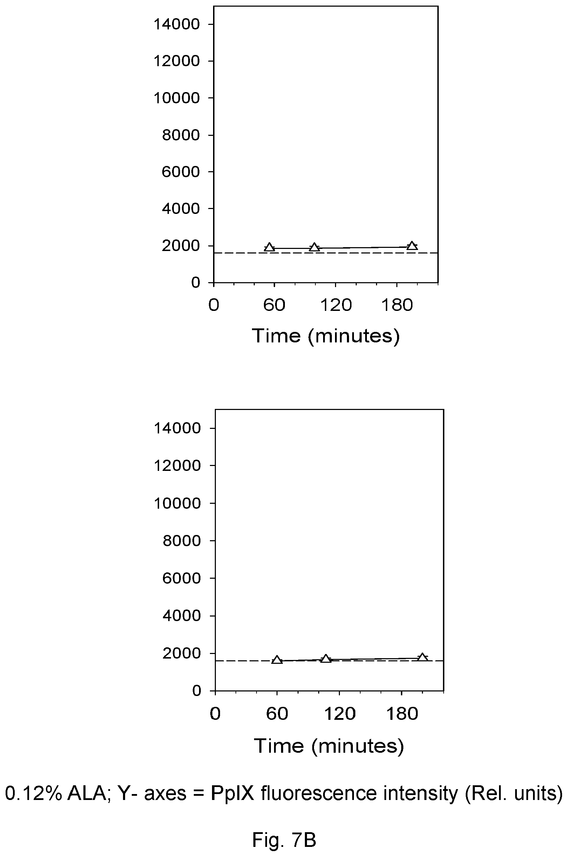

This example relates to the results shown in Figure 7 and relates to a test conducted with the La Roche-Posay Anthelios xl sunscreen including the ingredient ALA/PpIX 0.12% (w/w) applied to the facial skin of 6 volunteers for 3 hours, during which no ALA-PpIX fluorescent signals in the photodamaged and also normal skin areas of all volunteers were detected by spectrophotometry.

### Example 13

Comparison of skin improvement between 5-aminolevulinic acid alone and 5-aminolevulinic acid plus Vitamin D3 (Cholecalciferol) in a sunscreen product after daylight exposure.

Aim of the study: The aim of the study was to investigate the effect on human skin of low ("sub-therapeutic") concentrations of ALA alone or in combination with vitamin D3 in a commercial sunscreen product

### Study design:

### Products

(a) A commercial FPS-50 sunscreen product (La Roche-Posay Anthelios xl) supplemented with ALA (0.2 wt%)
(b) A commercial FPS-50 sunscreen product (La Roche-Posay Anthelios xl) supplemented with ALA (0.2 wt%) plus vitamin D3 (cholecalciferol) (0.0001 wt%)
(c) A commercial FPS-50 sunscreen product (La Roche-Posay Anthelios xl, Actinica Galderma 50+, or Cosmica 50) without any modifications.

### Selection of subjects

Subjects all signed an informed consent agreement before the study started.

### Ethic approvals

The Regional Ethic Committee and National Medicinal Authority both regarded the study as a non-pharmaceutical study due to the low concentrations of the active substances.

### Application of compositions

Topical application of the compositions to each side of the face was made daily for 6-12 weeks followed by daylight exposure. All subjects recorded the frequency of the use of the creams and daylight exposure and only subjects using the creams and daylight at least 5 days per week were included in this study.

### Examination

A survey was performed and the subjects reported daily the subjective and objective effects and side effects on their facial skin during the first week and weekly thereafter. No side-effects were reported by any of the subjects.

### Example 13 A

The effect of the composition on Actinic Keratosis (AK) was assessed. AK is an erythematous lesion of the skin with a fine or thick scale. Clinical and photographic assessments of AK were made before and after the daily use of the creams for 6-12 weeks.

Clinical grading of erythema (E): Grade 0: no erythema; Grade 1: pink; Grade 2: moderate redness; Grade 3: intense redness.

Clinical Grading of AK according to the Olsen classification 1991 (T)^{1.2}:
Grade 0: no actinic keratosis; Grade 1: single or few lesions, better felt than seen; Grade 2: moderate thick lesions (hyperkeratotic), easily felt and seen; Grade 3: thick hyperkeratotic lesions.

The clinical evaluation was performed independently by two experienced senior dermatologists, one by clinical examination on the subjects; while the other (blinded) on photographs only. Their evaluation is represented in table 1.

In addition, a specialised ultrasound device (Derma Lab, Skin Lab Combo, Cortex Technology, 9560 Hadsund, Denmark) was employed to measure the thickness, intensity and elasticity in the bilateral temple areas of the skin.

### Results and Conclusions:

**Table 1. Clinical evaluation of the skin improvement**

| | Right side 0.2% ALA | | Left side 0.2% ALA + vit D3 | |
|---|---|---|---|---|
| # test subject | **Before** | **After** | **Before** | **After** |
| No 1 | E 2 | E 1.5 | E 2.5 | E 1 |
| | T 2 | T 2 | T 2 | T 1 |
| No 2 | E 2.5 | E 2 | E 1.5 | E 0 |
| | T 2 | T 2 | T 1 | T 0 |
| No 3 | E 0 | E 0 | E 1.5 | E 1 |
| | T 0 | T 0 | T 2 | T 1 |
| No 4 | E 2 | E 1 | E 1 | E 1 |
| | T 2 | T 2 | T 2 | T 1 |

| | | | | |
|---|---|---|---|---|
| Note: T = Thickness (Olsen) grade 1-3; E = Erythema grade 1-3 Conclusion: ALA plus vitamin D3 was superior as compared to ALA alone regarding both reduction of erythema and thickness of AK. | | | | |

### Example 13B

The effect of the compositions on cosmetic factors such as skin thickness and elasticity was assessed by Dermalab equipment. Visco Elasticity (VE) represents a parameter where both the elevation phase and the retraction phase are taken into account.
VE = Young's modulus/ R normalized where R normalized = R/260 ms
VE with the unit MPa (Mega Pascal) can be measured by the Derma Lab ultrasound device. A reduction of VE represents an increase in the skin elasticity.

**Table 2. Ultrasound measurements of the skin elasticity before and after cream use and presented as difference in percentage**

| | Thickness of dermis | | Elasticity (VE) | |
|---|---|---|---|---|
| #test subject | Right side | Left side | Right side | Left side |
| | 0.2% ALA | ALA 0.2% + vit D3 | 0.2% ALA | ALA 0.2% + vit D3 |
| No 1 | -5 % | + 18 % | -44 % | -64 % |
| No 2 | -21 % | +25% | +16 % | -45 % |
| No 3 | +8 % | +23 % | 0 % | -62 % |
| No 4 | +6 % | + 19 % | -17 % | -17 % |

| | | | | |
|---|---|---|---|---|
| Conclusion: The composition ALA + vitamin D3 is superior as compared to ALA alone regarding the increase in thickness of the dermis, as well as increase in elasticity of the skin (indicated by a negative VE). | | | | |

### References:

1. Primary Care Dermatology Society (PCDS) guidelines 2012
2. De Berker D et al. British Journal of Dermatology 2007; 158:222-230

## Claims

1. A composition for use in treating or preventing skin damage from ultraviolet radiation, said composition comprising:
(a) 5-aminolevulinic acid (5-ALA) or an acid addition salt thereof;
(b) vitamin D3; and
(c) a UVA and/or UVB protective agent,
wherein said 5-ALA or acid addition salt thereof is present in an amount of 0.1 to 2.0 wt% of the composition, and wherein said vitamin D3 is present in an amount of 0.0001 to 0.005 wt% of the composition.

2. The composition for use of claim 1, wherein said composition is for use in treating or preventing skin damage from ultraviolet radiation and one or more signs of skin ageing, dryness, scarring and/or blemishes.

3. The composition for use of claim 2, wherein the one or more signs of skin ageing are selected from the group consisting of fine lines, wrinkles, reduced elasticity, reduced density, reduced firmness, reduced colour evenness, increased surface texture coarseness, discolouration, hyperpigmentation and/or increased mottled pigmentation.

4. The composition for use of claim 2, wherein the one or more signs of skin dryness are selected from the group consisting of a feeling of skin tightness, skin flaking, skin scaling, skin cracks, and/or skin redness.

5. The composition for use of claim 2, wherein the skin scarring or blemishes comprises a keloid scar, a hypertrophic scar, a contracture scar, a pitted scar or and/or a grooved scar.

6. The composition for use of any one of claims 1 to 5, wherein said 5-ALA or an acid addition salt thereof is present in an amount of 0.1 to 1.0 wt% of the composition.

7. The composition for use of claim 6, wherein said 5-ALA or an acid addition salt thereof is present in an amount of 0.1 to 0.5 wt% of the composition.

8. The composition for use of any one of claims 1 to 5, wherein said 5-ALA or an acid addition salt thereof is present in an amount of 1 to 2 wt% of the composition.

9. The composition for use of any one of claims 1 to 8, wherein said 5-ALA acid addition salt is the hydrochloride salt of 5-aminolevulinic acid (ALA-HCl).

10. The composition for use of any one of claims 1 to 9, wherein said composition comprises one or more skin conditioning agents, preservatives, skin penetration enhancers, thickeners, viscosity modifying agents, gelling agents, sequestering agents, pH adjusting agents, waxes, drying agents, anti-itch agents, anti-foaming agents, buffers, neutralizing agents, colouring agents, discolouring agents, emulsifying agents, emulsion stabilizers, odorants, antioxidants, diluents, carriers, and/or propellants.

11. The composition for use of any one of claims 1 to 10, wherein the composition is in the form of a lotion, salve, cream, gel, ointment, stick, spray, film, aerosol, drop, foam, mousse, solution, emulsion, suspension, dispersion, milk or patch.

12. The composition for use of any one of claims 1 to 11, wherein the composition is a sunscreen composition.

13. A kit comprising:
(a) 5-aminolevulinic acid (5-ALA) or an acid addition salt thereof;
(b) vitamin D3; and
(c) a UVA and/or UVB protective agent,
wherein said kit comprises a container comprising a composition comprising said 5-ALA or acid addition salt thereof in an amount of 0.1 to 2.0 wt% of the composition, and a separate container comprising a composition comprising said vitamin D3 present in an amount of 0.0001 to 0.005 wt% of the composition.

## Patentansprüche

1. Zusammensetzung zur Verwendung bei der Behandelung oder Vorbeugung von Hautschäden durch Ultraviolettstrahlung, wobei die Zusammensetzung umfasst:
(a) 5-Aminolevulinsäure (5-ALA) oder ein Säureadditionssalz davon;
(b) Vitamin D3; und
(c) ein UVA- und/oder UVB-Schutzmittel,
wobei die 5-ALA oder das Säureadditionssalz davon in einer Menge von 0,1 bis 2,0 Gew.-% der Zusammensetzung vorliegt und wobei das Vitamin D3 in einer Menge von 0,0001 bis 0,005 Gew.-% der Zusammensetzung vorliegt.

2. Zusammensetzung zur Verwendung nach Anspruch 1, wobei die Zusammensetzung zur Verwendung bei der Behandelung oder Vorbeugung von Hautschäden durch Ultraviolettstrahlung und einem oder mehreren Zeichen von Hautalterung, Trockenheit, Vernarbung und/oder Unregelmäßigkeiten dient.

3. Zusammensetzung zur Verwendung nach Anspruch 2, wobei das eine oder die mehreren Zeichen von Hautalterung ausgewählt sind aus der Gruppe bestehend aus feinen Linien, Falten, verringerter Elastizität, verringerter Dichte, verringerter Festigkeit, verringerter Farbgleichmäßigkeit, vergröberter Oberflächenstruktur, Verfärbungen, Hyperpigmentierung und/oder verstärkter marmorierter Pigmentierung.

4. Zusammensetzung zur Verwendung nach Anspruch 2, wobei das eine oder die mehreren Zeichen von Hauttrockenheit ausgewählt sind aus der Gruppe bestehend aus einem Spannungsgefühl der Haut, Hautschuppung, Schuppenbildung, Hautrissen und/oder Hautrötungen.

5. Zusammensetzung zur Verwendung nach Anspruch 2, wobei die Hautvernarbung oder -unregelmäßigkeiten eine Keloidnarbe, eine hypertrophe Narbe, eine kontrakturierte Narbe, eine löchrige Narbe oder/und eine gefurchte Narbe umfasst.

6. Zusammensetzung zur Verwendung nach einem der Ansprüche 1 bis 5, wobei die 5-ALA oder ein Säureadditionssalz davon in einer Menge von 0,1 bis 1,0 Gew.-% der Zusammensetzung vorliegt.

7. Zusammensetzung zur Verwendung nach Anspruch 6, wobei die 5-ALA oder ein Säureadditionssalz davon in einer Menge von 0,1 bis 0,5 Gew.-% der Zusammensetzung vorliegt.

8. Zusammensetzung zur Verwendung nach einem der Ansprüche 1 bis 5, wobei die 5-ALA oder ein Säureadditionssalz davon in einer Menge von 1 bis 2 Gew.-% der Zusammensetzung vorliegt.

9. Zusammensetzung zur Verwendung nach einem der Ansprüche 1 bis 8, wobei das 5-ALA-Säureadditionssalz das Hydrochloridsalz von 5-Aminolevulinsäure (ALA-HCl) ist.

10. Zusammensetzung zur Verwendung nach einem der Ansprüche 1 bis 9, wobei die Zusammensetzung ein oder mehrere Hautpflegestoffe, Konservierungsstoffe, Hautgängigkeitsverstärker, Dickungsstoffe, Viskositätsmodifikationsstoffe, Gelierstoffe, Sequestrierstoffe, pH-Einstellungsmittel, Wachse, Trocknungsstoffe, Antijuckstoffe, Antischaumstoffe, Pufferstoffe, Neutralisierungsstoffe, Farbstoffe, Entfärbungsstoffe, Emulgatoren, Emulsionsstabilisatoren, Geruchsstoffe, Antioxidantien, Verdünner, Träger und/oder Treibgase umfasst.

11. Zusammensetzung zur Verwendung nach einem der Ansprüche 1 bis 10, wobei die Zusammensetzung in Form einer Lotion, Salbe, Creme, eines Gels, einer Salbe, eines Stifts, eines Sprays, eines Films, eines Aerosols, eines Tropfens, eines Schaums, einer Mousse, einer Lösung, einer Emulsion, einer Suspension, einer Dispersion, einer Milch oder eines Pflasters vorliegt.

12. Zusammensetzung zur Verwendung nach einem der Ansprüche 1 bis 11, wobei die Zusammensetzung eine Sonnenschutzzusammensetzung ist.

13. Kit, umfassend:
(a) 5-Aminolevulinsäure (5-ALA) oder ein Säureadditionssalz davon;
(b) Vitamin D3; und
(c) ein UVA- und/oder UVB-Schutzmittel,
wobei das Kit einen Behälter, der eine Zusammensetzung umfasst, die die 5-ALA oder deren Säureadditionssalz in einer Menge von 0,1 bis 2,0 Gew.-% der Zusammensetzung umfasst, und einen separaten Behälter, der eine Zusammensetzung umfasst, die das Vitamin D3 umfasst, das in einer Menge von 0,0001 bis 0,005 Gew.-% der Zusammensetzung vorliegt, umfasst.

## Revendications

1. Composition destinée à être utilisée dans le traitement ou la prévention de lésions cutanées provenant d'un rayonnement ultraviolet, ladite composition comprenant :
(a) de l'acide 5-aminolévulinique (5-ALA) ou un sel d'addition d'acide de celui-ci ;
(b) de la vitamine D3 ; et
(c) un agent de protection contre les UVA et/ou les UVB,
dans laquelle ledit 5-ALA ou sel d'addition d'acide de celui-ci est présent en une quantité de 0,1 à 2,0 % en poids de la composition et dans laquelle ladite vitamine D3 est présente en une quantité de 0,0001 à 0,005 % en poids de la composition.

2. Composition destinée à être utilisée selon la revendication 1, dans laquelle ladite composition est destinée à être utilisée dans le traitement ou la prévention de lésions cutanées provenant d'un rayonnement ultraviolet et d'une ou de plusieurs signes de vieillissement, de sécheresse, de cicatrisation de la peau et/ou taches.

3. Composition destinée à être utilisée selon la revendication 2, dans laquelle les un ou plusieurs signes de vieillissement de la peau sont sélectionnés dans le groupe constitué par de fines lignes, des rides, une élasticité réduite, une densité réduite, une fermeté réduite, une répartition de couleur réduite, une rugosité de texture de surface accrue, une décoloration, une hyperpigmentation et/ou une pigmentation marbrée accrue.

4. Composition destinée à être utilisée selon la revendication 2, dans laquelle les un ou plusieurs signes de sécheresse de la peau sont sélectionnés dans le groupe constitué par un sentiment de fermeté de la peau, d'exfoliation de la peau, de desquamation de la peau, de craquelures de la peau et/ou de rougeur de la peau.

5. Composition destinée à être utilisée selon la revendication 2, dans laquelle la cicatrisation de la peau ou des taches comprennent une cicatrice chéloïde, une cicatrice hypertrophique, une cicatrice de contracture, une cicatrice grêlée et/ou une cicatrice rainurée.

6. Composition destinée à être utilisée selon l'une quelconque des revendications 1 à 5, dans laquelle ledit 5-ALA ou un sel d'addition d'acide de celui-ci est présent en une quantité de 0,1 à 1,0 % en poids de la composition.

7. Composition destinée à être utilisée selon la revendication 6, dans laquelle ledit 5-ALA ou un sel d'addition d'acide de celui-ci est présent en une quantité de 0,1 à 0,5 % en poids de la composition.

8. Composition destinée à être utilisée selon l'une quelconque des revendications 1 à 5, dans laquelle ledit 5-ALA ou un sel d'addition d'acide de celui-ci est présent en une quantité de 1 à 2 % en poids de la composition.

9. Composition destinée à être utilisée selon l'une quelconque des revendications 1 à 8, dans laquelle ledit sel d'addition d'acide 5-ALA est le sel de chlorhydrate de l'acide 5-aminolévulinique (ALA-HCl).

10. Composition destinée à être utilisée selon l'une quelconque des revendications 1 à 9, dans laquelle ladite composition comprend un ou plusieurs agents de conditionnement de la peau, conservateurs, amplificateurs de pénétration de la peau, épaississants, agents de modification de la viscosité, agents gélifiants, agents séquestrants, agents de réglage de pH, cires, agents de séchage, agents anti-puces, agents antimousse, tampons, agents neutralisants, agents colorants, agents décolorants, agents émulsifiants, agents de stabilisation d'émulsifon, agents odorisants, antioxydants, diluants, véhicules et/ou propulseurs.

11. Composition destinée à être utilisée selon l'une quelconque des revendications 1 à 10, dans laquelle la composition est sous la forme d'une lotion, d'un baume, d'une crème, d'un gel, d'un onguent, d'un bâton, d'un spray, d'un film, d'un aérosol, d'une goutte, d'une écume, d'une mousse, d'une solution, d'une émulsion, d'une suspension, d'une dispersion, d'un lait ou d'un timbre.

12. Composition destinée à être utilisée selon l'une quelconque des revendications 1 à 11, dans laquelle la composition est une composition d'écran solaire.

13. Kit comprenant :
(a) de l'acide 5-aminolévulinique (5-ALA) ou un sel d'addition d'acide de celui-ci ;
(b) de la vitamine D3 ; et
(c) un agent de protection contre les UVA et/ou les UVB,
dans lequel ledit kit comprend un récipient comprenant une composition comprenant ledit acide 5-ALA ou un sel d'addition d'acide de celui-ci en une quantité de 0,1 à 2,0 % en poids de la composition et un récipient distinct comprenant une composition comprenant ladite vitamine D3 présente en une quantité de 0,0001 à 0,005 % en poids de la composition.
